# EUROPEAN PATENT APPLICATION

(11) **EP 2 659 836 A1**
(43) Date of publication of application: **06.11.2013**
(21) Application number: 13166010.2
(22) Date of filing: 30.04.2013
(51) Int. Cl.: A61B 6/00, H02J 7/35

(54) **Solar powered wireless control device for medical imaging system**

(30) Priority: 02.05.2012 IN CH16992012
(71) Applicant: General Electric Company, Schenectady, New York 12345 (US)
(72) Inventor: Anand, Pradeep Kumar, 500056 Karnataka (IN); Siva, Prasad Bhagavatula Venkata, 500056 Karnataka (IN); Moore, Kyle, Salt Lake City, UT Utah 84116 (US)
(74) Representative: Illingworth-Law, William Illingworth

(57) **Abstract**

A control device for controlling the operation of a medical imaging system 300 is disclosed. The control device comprises multiple solar energy units 102 capable of generating electrical energy from ambient light energy. The control device also comprises multiple switches 104,106 connected to the multiple solar energy units 102 and capable of receiving the electrical energy for operating the medical imaging system 300.

## Description

The subject matter disclosed herein relates to a control device used for operating a medical imaging system. More specifically relates to, a battery charging unit of a control device.

Medical imaging systems are used for capturing images of human body parts for clinical purposes such as, medical examination or diagnosis of any disease. Various types of medical imaging systems exist such as but not limited to, a C-arm imaging system, an X-ray imaging system, a magnetic resonance imaging system, a dental imaging system and a computed tomography imaging system. These medical imaging systems are used for different types of surgery or diagnosis based on the type of disease.

Most of these medical imaging systems are operated in a confined location such as but not limited to operation theatres and laboratories by doctors, lab technicians and medical experts. During operation of a medical imaging system, in order to take an image of a patient's body part an image capturing unit needs to be aligned with the patient's body part. Then a control device including a hand switch or foot switch may be operated by the user to capture the image of the body part. The hand switch or foot switch may be operated by a foot or a hand of an operator respectively. The control device may be connected to the medical imaging system. The connection may be a wired or a wireless connection. Multiple images of the body part may be captured by operating the control device. The control device requires power that is supplied from a battery unit. The battery unit used commonly may be a replaceable battery or a rechargeable battery. A battery unit in the case of the wireless control device may be charged by the power supplied by the medical imaging system. Whereas a battery unit of a wireless control device may be charged by an external charger. In another scenario a battery unit may need to be docked in the medical imaging system for charging. So there may be some instances when the wireless control device may not have enough power available while in use causing inconvenience of operation. Further the user may need to remember and keep the wireless control device to be charged by the external charger or by docking inside the medical imaging system. There may be instances when the user may forget to put the wireless control device for charging causing inconvenience.

Thus there is a need for a battery charging unit that can be conveniently charged and used for supplying power to the control device without any dependencies to a medical imaging system or a power source present in the wall.

The above-mentioned shortcomings, disadvantages and problems are addressed herein which will be understood by reading and understanding the following specification.

In an embodiment a control device for controlling the operation of a medical imaging system is disclosed. The control device comprises multiple solar energy units capable of generating electrical energy from ambient light energy. The control device also comprises multiple switches connected to the at least one solar energy unit and are capable of receiving the electrical energy for operating the medical imaging system.

In another embodiment a medical imaging system comprises an image processor and a control device. The image processor processes an image of a body part of a patient. The control device is capable of sending signals to the image processor. These signals are for instructing the image processor to capture the image. The control device includes one or more solar energy units that are capable of generating electrical energy from ambient light energy. The control device also includes multiple switches that are capable of receiving the electrical energy from the solar energy units.

Various other features, objects, and advantages of the invention will be made apparent to those skilled in the art from the accompanying drawings and detailed description thereof.
FIGURE 1 is a schematic illustration of a control device having a solar energy unit in accordance with an embodiment;
FIGURE 2 is a schematic illustration of the solar energy unit illustrated in FIGURE 1 in accordance with an embodiment.
FIGURE 3 is a schematic illustration of a medical imaging system connected to the control device illustrated in FIGURE 1 in accordance with an embodiment; and
FIGURE 4 is a schematic illustration of a block diagram of a medical imaging system having a wireless communication with a control device.

In the following detailed description, reference is made to the accompanying drawings that form a part hereof, and in which is shown by way of illustration specific embodiments that may be practiced. These embodiments are described in sufficient detail to enable those skilled in the art to practice the embodiments, and it is to be understood that other embodiments may be utilized and that logical, mechanical, electrical and other changes may be made without departing from the scope of the embodiments. The following detailed description is, therefore, not to be taken as limiting the scope of the invention.

As discussed in detail below, embodiments of the invention include a control device and a medical imaging system for capturing an image of a body part of a patient. The medical imaging system comprises an image processor and a control device. The image processor processes an image of a body part of a patient. The control device is capable of sending signals to the image processor. These signals are for instructing the image processor to capture the image. The control device includes one or more solar energy units that are capable of generating electrical energy from ambient light energy. The control device also includes multiple switches that are capable of receiving the electrical energy from the solar energy units.

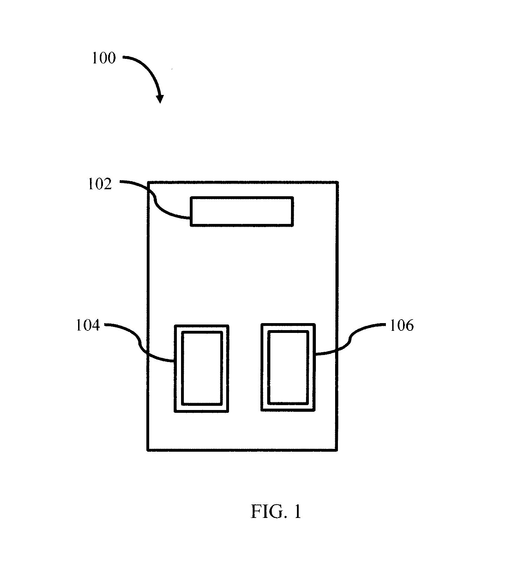

FIG. 1 is a schematic illustration of a control device 100 having a solar energy unit 102 in accordance with an embodiment. The control device 100 may be used for controlling the operation of a medical imaging system (not shown in FIG. 1). The medical imaging system may include but not limited to a C-arm imaging system, an X-ray imaging system, a magnetic resonance imaging system, a dental imaging system and a computed tomography imaging system. The control device 100 may be a wireless control device or a wired control device. The control device 100 may include multiple switches such as, a switch 104 and a switch 106 used for operating the medical imaging system. The switch104 may be activated for capturing an image of a body part of a patient. The switch 106 may be used for another function such as but not limited to printing the captured image and storing the captured image. However it may be appreciated that the control device 100 may include more switches that are used to perform other functionalities known in the art.

In an embodiment, switches such as the switch 104 and the switch 106 present in the control device 100 may be operated by a foot of user. In this scenario the control device 100 acts as a foot switch device. The switches in the foot switch device may be in form of pedals that may be operated by the user's foot. In another embodiment, switches such as the switch 104 and the switch 106 present in the control device 100 may be operated by a hand of the user. In this case the control device 100 acts as a hand switch device including switches operable by the user's hand.

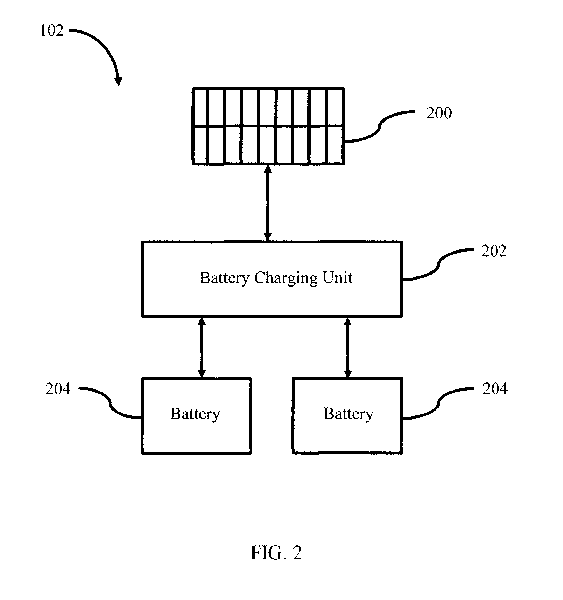

The control device 100 requires power for operation provided by the solar energy unit 102. The solar energy unit 102 may be embedded in the control device 100. The solar energy unit 102 receives ambient light energy and generates electric energy using the received light energy. The ambient light energy may be received from any light source in the location where the control device 100 may be placed. The light source may be natural light source or any other light source. For example, a solar energy unit of a control device present in an operation theatre receives light energy from light sources present in the operation theatre. The operation theatre may have enough light throughout a day. Thus solar energy unit may receive light energy throughout the day for generating the electric energy. As a result the control device 100 is constantly charged with power. The control device 100 may include multiple solar energy units similar to the solar energy unit 102. FIG. 2 illustrates the solar energy unit 102 in accordance with an embodiment. The solar energy unit 102 includes a solar panel 200, a battery charging unit 202 and one or more batteries 204. The solar panel 200 includes multiple solar cells that receive the ambient light energy and stores the light energy. The stored light energy may be then converted into electric energy. The light energy may be converted into electric energy using the techniques known in the art. In an embodiment a solar panel such as the solar panel 200 may be capable of generating electric energy of 0.8 Watts. The solar panel may have a dimension such as, a length of about 4.5 inches, a breadth of about 3.5 inches and a thickness of about 0.5 inches. Further the weight of the solar panel may be about 5.5 oz. However it may be appreciated that the solar panel may have any other dimensions and configuration that enables the solar panel to generate and supply power for the control device to operate.

The electric energy generated by the solar panel 200 is then accumulated or stored in the battery 204 through battery charging unit 202. The battery charging unit 202 is electrically connected to the solar panel 200 for receiving the electric energy. In an embodiment multiple battery charging units may be present in the solar energy unit 102. The battery charging unit 202 may be electrically connected to the batteries 204 and supply the stored electrical energy to the batteries 204 to charge them. The stored electric energy is utilized for operation of the control device 100. In an embodiment a battery may have a capacity varying from about 1000 milliampere hour (mAh) to about 2000 mAh. However batteries having any energy capacities may be used in the solar energy unit 100. The capacity of batteries and capacity of the solar panel 200 may be dependent on various factors such as but not limited to, number of switch presses per day, time for each switch press, total average imaging (such as, fluoroscopy) time per diagnostic or examination procedure, number of imaging per procedure, imaging time per procedure, control device's current consumption during an active mode and a sleep mode, transition time from the active mode to the sleep mode, total time for which the control device is in the active mode, total time for which the control device is in the passive mode and life of the medical imaging system.

The solar panel 200 can generate more electric energy when more light energy is available. For example when the light energy available to the solar panel 200 is about 200 luminous flux (lux) then the battery may be charged in about 12.5 hours. Further when available light energy ranges from about 40,000 lux to about 150,000 lux then the battery may be charged much faster. As the solar energy unit 102 uses ambient light energy available almost throughout the day the solar energy unit 102 charges battery 104 throughout the day and maintain its charge level. Thus the solar energy unit 102 can continuously supply power for charging the batteries 204 resulting in convenient operation of the control device 100 without any interruption or dependencies to any other power source. Further the solar energy unit 102 also avoids the need of placing the control device 100 for charging batteries in docking system of medical imaging system or by supplying power from a power source present in the wall.

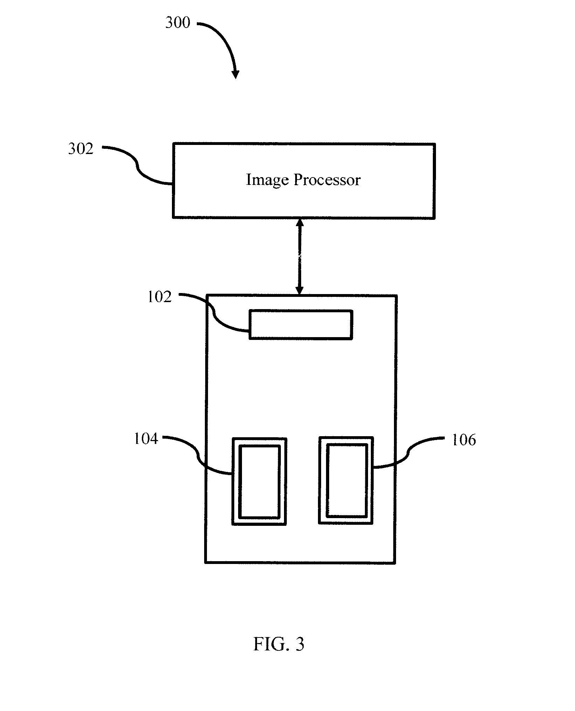

Turning now to FIG. 3, FIG. 3 illustrates a medical imaging system 300 including the control device 100 of FIG. 1 connected to an image processor 302 in accordance with an embodiment. The image processor 302 receives signals from the control device 100 for capturing the image of the body part of the patient. For example a patient may be positioned with respect to the medical imaging system 300. Then a body part of the patient that needs to be diagnosed is determined by an operator. The operator then presses a switch such as the switch104 for sending signals to the image processor 302 for capturing the image of the selected body part. The control device 100 uses the power generated by the solar energy unit 102 for operation as described in detail in conjunction with FIG. 1 and FIG. 2. The image processor 302 sends instructions to an image capturing unit 302 to capture the image. The image capturing unit may be a hardware unit that takes the image of the body part and then sends to the image processor 302. The image processor 302 then processes the image. The image may be processed using any techniques known in the art for presenting it to the operator. The operator may use the switch 106 for storing the captured image. Further the control device 100 may also include other switches that may be operated to send signals to the medical imaging system 300 for printing the image or for any other activity. The communication between the control device 100 and the medical imaging system 300 may be over a wireless communication link.

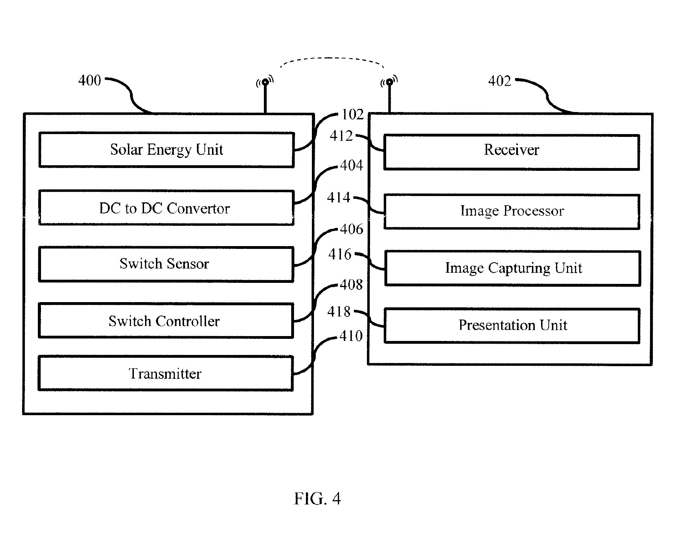

Referring to FIG. 4, FIG. 4 is a schematic illustration of a block diagram of a control device 400 having a wireless communication with a medical imaging system 402. The control device 400 and the medical imaging system 402 may be similar to the control device 100 and the medical imaging system 300 respectively. The control device 400 includes the solar energy unit 102, a DC to DC convertor 404, a switch sensor 406, a switch controller 408, and a transmitter 410. The solar energy unit 102 generates the electric energy using the ambient light energy. The electric energy is used for charging batteries such as batteries 204 of the control device 400. This is explained in conjunction with FIGs. 1 and 2. The electric energy stored is supplied to the switch sensor 406, the switch controller 408, and the transmitter 410. The switch sensor 406, the switch controller 408, and the transmitter 410 may have different power requirements.

In order to meet the power requirements initially the electric energy is converted into different voltages by the DC to DC convertor 404 based on the power requirements. The different voltages may be then supplied to the switch sensor 406, the switch controller 408 and the transmitter 410 based on their power requirements. The switch sensor 406 receives signals from a switch such as, the switch 104 and the switch 106. The switch sensor 406 sends these signals such as, control signals to the switch controller 408. The switch controller 408 processes the signals to generate instruction signals that may be understandable to the medical imaging system 402. The instruction signals may be transmitted to the medical imaging system 402 through the transmitter 410.

The transmitted instruction signals are received by a receiver 412 of the control device 402. The instruction signals may include but not limited to signals for capturing the image, storing the image and printing the captured image. The instruction signal for capturing the image is processed by an image processor 414 for capturing the image of the body part of the patient. The image processor 414 instructs an image capturing unit 416 for capturing the image. The captured image is then processed by the image processor 414 for display to the operator or a medical expert through a presentation unit 418. The presentation unit 418 may be a hardware monitor or a user interface of the medical imaging system 402. The medical imaging system 402 may include other units for showing a real time video of the body of part of the patient through the presentation unit 418.

This written description uses examples to disclose the invention, including the best mode, and also to enable any person skilled in the art to practice the invention, including making and using any computing system or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

## Claims

1. A control device (100) for controlling the operation of a medical imaging system (300), the control device comprises:
at least one solar energy unit (102) capable of generating electrical energy from ambient light energy; and
at least one switch (104,106) connected to the at least one solar energy unit (102) and capable of receiving the electrical energy for operating the medical imaging system.

2. The control device of claim 1, wherein a solar energy unit of the at least one solar energy unit comprises:
a solar panel for receiving the ambient light energy and converting into electrical energy;
a battery charging unit for accumulating the electrical energy; and
at least one battery connected to the battery charging unit and capable of storing electrical energy.

3. The control device of claim 1 or claim 2, wherein the control device is a wireless control device.

4. The control device of any preceding claim, wherein in the at least one switch comprises a foot switch.

5. The control device of any preceding claim, wherein the at least one switch comprises a hand switch.

6. The control device of any preceding claim, wherein the medical imaging system is one of a C-Arm imaging system, a dental imaging system and X-Ray imaging system.

7. A medical imaging system comprising:
an image processor for processing an image of a body part of a patient; and
a control device of any preceding claim capable sending signals to the image processor for processing the image.

8. A medical imaging system comprising:
an image processor for processing an image of a body part of a patient; and
a control device capable of sending signals to the image processor for processing the image, wherein the control device comprises;
at least one solar energy unit capable of generating electrical energy from ambient light energy; and
at least one switch connected to the at least one solar energy unit and capable of receiving the electrical energy from the at least one solar energy unit.

9. The medical imaging system of claim 7 or claim 8, wherein a solar energy unit of the at least one solar energy unit comprises:
a solar panel for receiving the ambient light energy and converting into electrical energy;
a battery charging unit for accumulating the electrical energy; and
at least one battery connected to the battery charging unit and capable of storing the electrical energy.

10. The medical imaging system of any of claims 7 to 9, wherein the control device is a wireless control device.

11. The medical imaging system of any of claims 7 to 10, wherein in the at least one switch comprises a foot switch.

12. The medical imaging system of any of claims 7 to 11, wherein the at least one switch comprises a hand switch.

13. The medical imaging system of any of claims 7 to 12, wherein the medical imaging system is one of a C-Arm imaging system, a dental imaging system and X-Ray imaging system.
